Europäisches Patentamt

European Patent Office

Office européen des brevets

(19)

(11) **EP 1 082 953 A1**

(12) # DEMANDE DE BREVET EUROPEEN

(43) Date de publication:
**14.03.2001 Bulletin 2001/11**

(51) Int Cl.$^{7}$: **A61K 7/032**, A61K 7/021

(21) Numéro de dépôt: **00402259.6**

(22) Date de dépôt: **09.08.2000**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **07.09.1999 FR 9911173**

(71) Demandeur: **L'OREAL**
**75008 Paris (FR)**

(72) Inventeur: **de la Poterie, Valérie**
**77820 Le Châtelet-en-Brie (FR)**

(74) Mandataire: **Kromer, Christophe**
**L'OREAL - D.P.I.,**
**6, rue Bertrand Sincholle**
**92585 Clichy Cedex (FR)**

(54) **Composition cosmétique comprenant des dispersions de particules de polymère dans une phase grasse liquide**

(57) L'invention a pour objet une composition à application topique comprenant une dispersion de particules dans une phase grasse liquide d'un premier système polymérique filmifiable et une dispersion de particules dans la phase grasse liquide d'un deuxième système polymérique non filmifiable.

L'invention a aussi pour objet un procédé cosmétique de maquillage ou de traitement non thérapeutique des matières kératiniques comprenant l'application sur les matières kératiniques d'une telle composition.

La composition permet d'obtenir un film brillant et non collant, ayant des propriétés de "sans transfert" et assurant en particulier un bon recourbement des fibres kératiniques.

EP 1 082 953 A1

**Description**

La présente invention a pour objet une composition à application topique comprenant des particules de polymère dispersées dans une phase grasse liquide, destinée en particulier au domaine cosmétique. Plus spécialement, l'invention se rapporte à une composition pour le soin ou le maquillage des matières kératiniques telles que la peau, y compris les lèvres, et les phanères comme les cils, les sourcils, les cheveux, et les ongles, notamment d'êtres humains.

Cette composition peut se présenter sous la forme de mascara, d'eye-liner, de produit pour les lèvres, de fard à joues ou à paupières, de fond de teint, de produit de maquillage du corps, de produit anti-cernes, de base de maquillage, de composition à appliquer sur un maquillage - dit encore top-coat - , de composition de protection solaire, de coloration de la peau, de produit de soin de la peau.

Les produits de maquillage ou de soin de la peau ou des lèvres d'êtres humains comme les eye-liners, les fonds de teints, les rouges à lèvres, ou bien encore des cils comme les mascaras, contiennent généralement des corps gras tels que des cires et des huiles, des pigments et/ou charges et, éventuellement des additifs comme des actifs. Ces compositions sont généralement appliquées sur la peau ou les cils sous forme de couche mince conduisant à la formation d'un dépôt mince.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué, nécessitant de renouveler régulièrement l'application de la composition de fond de teint ou de rouge à lèvres. Par ailleurs, l'apparition de ces traces inacceptables notamment sur les cols de chemisier peut écarter certaines femmes de l'utilisation de ce type de maquillage.

Il est connu des documents EP-A-749746 et FR-A-2772600 des compositions cosmétiques comprenant des particules de polymère filmogène dispersées dans une phase grasse liquide et stabilisées en surface. Ces compositions, bien que présentant des propriétés de "sans transfert" améliorées, ont l'inconvénient de laisser sur les matières kératiniques un film peu brillant lorsque la composition comprend des matières pulvérulentes comme les charges.

La présente invention a donc pour but de proposer une composition ne présentant pas les inconvénients ci-dessus et conduisant, après application sur les matières kératiniques, à la formation d'un film brillant et non collant, tout en ayant des propriétés de "sans transfert".

Par ailleurs, personne jusqu'à ce jour n'a envisagé d'utiliser des particules de polymère dispersées et stabilisées en surface dans une phase grasse dans une composition de maquillage de fibres kératiniques tels que les cils pour leur gainage et/ou leur recourbement tout en ayant des propriétés de brillance et de non transfert, aisément démaquillable.

Les inventeurs ont découvert qu'une telle composition pouvait être obtenue en utilisant un premier système polymérique filmifiable et un deuxième système polymérique non filmifiable dispersés dans une phase grasse liquide.

Plus précisément, l'invention a pour objet une composition à application topique comprenant, dans un milieu physiologiquement acceptable, une dispersion de particules dans une phase grasse liquide d'un premier système polymérique filmifiable comprenant un premier polymère, stabilisées en surface, caractérisée par le fait qu'elle comprend, de plus, une dispersion de particules dans la phase grasse liquide d'un deuxième système polymérique non filmifiable comprenant un deuxième polymère, stabilisées en surface.

Par "milieu physiologiquement acceptable", il faut comprendre un milieu compatible avec les matières kératiniques, comme un milieu cosmétique.

L'invention a aussi pour objet un procédé cosmétique de maquillage ou de traitement non thérapeutique des matières kératiniques, comprenant l'application sur celles-ci d'une composition telle que décrite précédemment.

Un autre objet de l'invention est l'utilisation d'une composition telle que définie précédemment pour obtenir un film déposé sur les matières kératiniques, notamment les fibres kératiniques, brillant et/ou non collant et/ou ayant des propriétés de non transfert.

L'invention a encore pour objet l'utilisation d'une composition telle que définie précédemment pour recourber les fibres kératiniques, et notamment les cils.

La composition selon l'invention comprend une dispersion stable de particules généralement sphériques, dans une phase grasse liquide physiologiquement acceptable, d'un premier système polymérique filmifiable comprenant un premier polymère et d'un deuxième système polymérique non filmifiable comprenant un deuxième polymère. Les particules de chaque système polymérique ont de préférence une taille allant de 5 nm à 600 nm, étant donné qu'au-delà d'environ 600 nm, les dispersions de particules deviennent beaucoup moins stables, et de préférence de 50 nm à 250 nm.

Le système polymérique :

On entend par "système polymérique filmifiable" un système polymérique apte à former un film isolable à température ambiante (25 °C). Le premier système polymérique filmogène a de préférence une température de transition vitreuse (Tg 1) basse, inférieure ou égale à la température de la peau, et en particulier inférieure ou égale à environ 40 °C. Avantageusement, la Tg 1 va environ de -100 °C à environ 40 °C, et mieux allant de - 10 °C à 30 °C.
On entend par "système polymérique non filmifiable", un système polymérique qui ne permet pas de former un film isolable à température ambiante (25 °C). Le deuxième système polymérique non filmifiable permet toutefois, en association avec la phase grasse liquide, de former un dépôt continu et homogène sur les matières kératiniques. Le deuxième système polymérique non filmifiable a de préférence une température de transition vitreuse (Tg 2) supérieure à environ 40 °C, et en particulier est inférieure ou égale à 300 °C. Avantageusement, la Tg 2 va environ de 45 °C à 150 °C.
La mesure de Tg (température de transition vitreuse) est effectuée par DSC (Differential Scanning Calorimetry) selon la norme ASTM D3418-97.
Selon un premier mode de réalisation de la composition selon l'invention, le premier et/ou deuxième système polymérique, indépendamment l'un de l'autre, peut consister essentiellement, respectivement, en un ou plusieur(s) premier(s) et deuxième(s) polymère(s) ayant les caractéristiques du système polymérique correspondant décrites précédemment.
Lorsque le premier et/ou le deuxième polymère ne permettent pas d'obtenir seul les caractéristiques du système polymérique correspondant mentionnées précédemment, il est possible d'ajouter un composé dont la fonction est de modifier les propriétés de chaque polymère pour obtenir le système polymérique souhaité. Aussi, selon un second mode de réalisation de la composition selon l'invention, on peut ajouter au premier et/ou deuxième polymères au moins un agent plastifiant permettant d'obtenir un système polymérique ayant les caractéristiques telles que décrites précédemment. Dans ce cas, le ou les systèmes polymériques comprennent un mélange d'un ou de plusieurs polymères et d'au moins un agent plastifiant.
Le ou les plastifiants peuvent être choisis parmi les plastifiants usuellement utilisés dans le domaine d'application et notamment parmi les composés susceptibles d'être des solvants de l'un des polymères.
Les premier et deuxième polymères utilisés dans la présente invention peuvent être de toute nature. Parmi les polymères utilisables, on peut citer les polymères radicalaires, les polycondensats, et les polymères d'origine naturelle et leurs mélanges. Les polymères peuvent être choisis par l'homme du métier en fonction de leurs propriétés.
Les polymères utilisables dans la composition de l'invention ont de préférence un poids moléculaire moyen en poids allant de l'ordre de 2000 à 10 000 000.
Par polymère radicalaire, on entend un polymère obtenu par polymérisation d'un ou plusieurs monomères à insaturation notamment éthylénique, certain monomère étant susceptible de s'homopolymériser (à l'inverse des polycondensats). Les polymères radicalaires peuvent être notamment des polymères ou des copolymères, vinyliques, notamment des polymères acryliques.
Les polymères vinyliques peuvent résulter de la polymérisation de monomères à insaturation éthylénique ayant au moins un groupement acide et/ou des esters de ces monomères acides et/ou des amides de ces acides.
Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique et leurs associations. On utilise de préférence l'acide (méth)acrylique et l'acide crotonique, et plus préférentiellement l'acide (méth)acrylique.
Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{20}$, de préférence en $C_1$-$C_8$, des (méth)acrylates d'aryle, en particulier d'aryle en $C_6$-$C_{10}$, des (méth)acrylates d'hydroxyalkyle, en particulier d'hydroxyalkyle en $C_2$-$C_6$ .
Parmi les (méth)acrylates d'alkyle, on peut citer le (méth)acrylate de méthyle, le (méth)acrylate d'éthyle, le (méth)acrylate de butyle, le (méth)acrylate d'isobutyle, le (méth)acrylate d'éthyl-2 hexyle, le (méth)acrylate de lauryle.
Parmi les (méth)acrylates d'hydroxyalkyle, on peut citer le (méth)acrylate d'hydroxyéthyle, le (méth)acrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.
Comme polymère radicalaire, on utilise de préférence les copolymère d'acide (méth)acrylique et de (méth)acrylate d'alkyle, notamment d'alkyle en $C_1$-$C_4$. Plus préférentiellement, on peut utiliser les acrylates de méthyle éventuellement copolymérisés avec l'acide acrylique.
Comme amides des monomères acides, on peut citer les (méth)acrylamides, et notamment les N-alkyl (méth) acrylamides, en particulier d'alkyl en $C_2$-$C_{12}$ tels que le N-éthyl acrylamide, le N-t-butyl acrylamide, le N-octyl acrylamide ; les N- dialkyl ($C_1$-$C_4$) (méth)acrylamides.

Les polymères vinyliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyl, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styréniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle.

Comme monomères styréniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques, on peut encore citer :

- la N-vinylpyrrolidone; la vinylcaprolactame; les vinyl N-alkyl($C_1$-$C_6$) pyrroles; les vinyl-oxazoles; les vinyl-thiazoles; les vinylpyrimidines; les vinylimidazoles;

- les oléfines tels que l'éthylène, le propylène, le butylène, l'isoprène, le butadiène.

Le polymère vinylique peut être réticulé à l'aide de monomère difonctionnel, notamment comprenant au moins deux insaturations éthyléniques, tel que le diméthacrylate d'éthylène glycol, le phtalate de diallyle.

Avec de telles dispersions de particules de polymère, il est possible de calibrer à volonté la taille des particules de polymère, et de moduler leur "polydispersité" en taille lors de la synthèse. Il est ainsi possible d'obtenir des particules de très petite taille, qui sont invisibles à l'oeil nu lorsqu'elles sont dans la composition et lorsqu'elles sont appliquées sur la peau ou les lèvres. Ceci ne serait pas possible avec des pigments sous forme particulaire, leur constitution ne permettant pas de moduler la taille moyenne des particules.

De façon non limitative, les polymères de l'invention peuvent être choisis parmi, les polymères ou copolymères suivants : polyuréthannes, polyuréthannesacryliques, polyurées, polyurée-polyuréthannes, polyester-polyuréthannes, polyéther-polyuréthannes, polyesters, polyesters amides, polyesters à chaîne grasse, alkydes ; polymères siliconés, polymères fluorés et leurs mélanges.

Les premier et deuxième polymères dispersés sont utilisés en une quantité efficace pour obtenir un film continu et homogène, déposé sur les matières kératiniques et plus spécialement les fibres kératiniques, brillant et/ou non collant et/ou ayant des propriétés de non transfert.

En pratique, les premier et deuxième polymères dispersés dans la phase grasse liquide peuvent être présents en une teneur totale,allant de 2 % à 60 % en poids, par rapport au poids total de la composition, de préférence de 4 % à 40 % en poids, et mieux de 5 % à 30 % en poids.

Avantageusement, les premier et deuxième polymères peuvent être présents dans la composition selon un rapport pondéral premier polymère/deuxième polymère allant de 1/99 à 99/1, de préférence 50/50 à 99/1 et mieux de 50/50 à 80/20.

La phase grasse liquide :

Par "phase grasse liquide", on entend tout milieu non aqueux liquide à température ambiante (25 °C) et pression atmosphérique. Cette phase grasse peut contenir une phase grasse liquide volatile et/ou une phase grasse liquide non volatile contenant respectivement une ou plusieurs huiles liquides.

Par "phase grasse volatile", on entend tout milieu non aqueux susceptible de s'évaporer de la peau en moins d'une heure. Cette phase volatile comporte notamment des huiles ayant une pression de vapeur, à température ambiante et pression atmosphérique allant de $10^{-3}$ à 300 mm de Hg (0,13 Pa à 40 000 Pa).

La phase grasse liquide dans laquelle sont dispersés les premier et deuxième polymères, peut comprendre toute huile physiologiquement acceptable et en particulier cosmétiquement acceptable, notamment au moins une huile choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

La phase grasse liquide totale de la composition peut représenter de 10 % à 98 % en poids, par rapport au poids total de la composition et de préférence de 20 à 85 % en poids. La partie non volatile peut représenter de 0 à 80 % (notamment de 0,1 à 80 %) du poids total de la composition et mieux de 1 à 50 %.

Comme phase grasse liquide utilisable selon l'invention, on peut ainsi citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyldécyle, le palmitate de 2-octyl-décyle, le myristate ou le lactate de 2-octyldodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les polydiméthylsiloxane (PDMS), éventuellement phénylées telles que les phényltriméthicones, ou éventuellement substitués par des groupements aliphatiques et/ou aromatiques, éventuellement fluorés, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées.

Avantageusement, on peut utiliser une ou plusieurs huiles volatiles à température ambiante. Ces huiles volatiles sont favorables à l'obtention d'un film à propriétés "sans transfert" total, à savoir résistant totalement aux frottements. Après évaporation de ces huiles, on obtient un dépôt filmogène souple, non collant. Ces huiles volatiles facilitent, en outre, l'application de la composition sur les fibres kératiniques comme les cils.

Ces huiles volatiles peuvent être des huiles hydrocarbonées, des huiles siliconées comportant éventuellement des groupements alkyle ou alkoxy en bout de chaîne siliconée ou pendante.

Comme huile de silicone volatile utilisable dans l'invention, on peut citer les silicones linéaires ou cycliques ayant de 2 à 7 atomes de silicium et/ou une viscosité inférieure à 8 cst, ces silicones comportant éventuellement des groupes alkyle ou alkoxy ayant de 1 à 10 atomes de carbone. On peut citer notamment l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.

Comme huile hydrocarbonée volatile, on peut citer les isoparaffines en $C_8$-$C_{16}$ telles que les 'ISOPARs', les PERMETYLs et notamment l'isododécane.

Ces huiles volatiles peuvent être présentes dans la composition en une teneur allant de 5 à 98 % du poids total de la composition, et mieux de 30 à 75 %.

Dans un mode particulier de réalisation de l'invention, on choisit la phase grasse liquide dans le groupe comprenant :

- les composés liquides non aqueux ayant un paramètre de solubilité global selon l'espace de solubilité de HANSEN inférieur à 17 $(MPa)^{\frac{1}{2}}$,
- ou les monoalcools ayant un paramètre de solubilité global selon l'espace de solubilité de HANSEN inférieur ou égal à 20 $(MPa)^{\frac{1}{2}}$,
- ou leurs mélanges.

Le paramètre de solubilité global δ global selon l'espace de solubilité de HANSEN est défini dans l'article "Solubility parameter values" de Eric A. Grulke de l'ouvrage "Polymer Handbook" 3ème édition, Chapitre VII, pages 519-559 par la relation :

$$\delta = (d_D^2 + d_P^2 + d_H^2)^{1/2}$$

dans laquelle

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.). La définition des solvants dans l'espace de solubilité tridimensionnel selon HANSEN est décrite dans l'article de C. M. HANSEN : "The three dimensional solubility parameters" J. Paint Technol. 39, 105 (1967).

Parmi les phases grasses liquides ayant un paramètre de solubilité global selon l'espace de solubilité de HANSEN inférieur ou égal à 17 $(MPa)^{\frac{1}{2}}$, on peut citer des huiles végétales formées par des esters d'acides gras et de polyols, en particulier les triglycérides, telles que l'huile de tournesol, de sésame ou de colza, ou les esters dérivés d'acides ou d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle. On peut également citer les hydrocarbures et notamment des huiles de paraffine, de vaseline, ou le polyisobutylène hydrogéné, l'isododécane, ou encore les 'ISOPARs', isoparaffines volatiles. On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyle, thiol et/ou amine, et les huiles siliconées volatiles, notamment cycliques. On peut également citer les solvants, seuls ou en mélange, choisis parmi (i) les esters linéaires, ramifiés ou cycliques, ayant plus de 6 atomes de carbone, (ii) les éthers ayant plus de 6 atomes de carbone, (iii) les cétones ayant plus de 6 atomes de carbone. Par monoalcools ayant un paramètre de solubilité global selon l'espace de solubilité de HANSEN inférieur ou égal à 20 $(MPa)^{1/2}$, on entend les alcools gras aliphatiques ayant au moins 6 atomes de carbone. Comme monoalcools selon l'invention, on peut citer l'alcool oléique, le décanol, le dodécanol, l'octadécanol et l'alcool linoléique.

Comme milieu non aqueux, on peut aussi utiliser ceux décrits dans le document FR-A-2 710 646 de L.V.M.H.

Le choix du milieu non aqueux est effectué par l'homme du métier en fonction de la nature des monomères constituant les polymères et/ou de la nature du stabilisant, comme indiqué ci-après.

La composition selon l'invention est obtenue par mélange de la dispersion de particules du premier système polymérique filmifiable avec la dispersion de particules du deuxième système polymérique.

Chaque dispersion de polymère peut être fabriquée comme décrit dans le document EP-A-749747. La polymérisation peut être effectuée en dispersion, c'est-à-dire par précipitation du polymère en cours de formation, avec protection des particules formées avec un stabilisant.

On prépare donc un mélange comprenant les monomères initiaux ainsi qu'un amorceur radicalaire. Ce mélange est dissous dans un solvant appelé, dans la suite de la présente description, "solvant de synthèse". Lorsque la phase grasse est une huile non volatile, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc un solvant de synthèse tel que les monomères initiaux, et l'amorceur radicalaire, y sont solubles, et les particules de chaque polymère obtenu y sont insolubles afin qu'elles y précipitent lors de leur formation. En particulier, on peut choisir le solvant de synthèse parmi les alcanes tels que l'heptane, l'isododécane ou le cyclohexane.

Lorsque la phase grasse choisie est une huile volatile, on peut directement effectuer la polymérisation de chaque polymère dans ladite huile qui joue donc également le rôle de solvant de synthèse. Les monomères doivent également y être solubles, ainsi que l'amorceur radicalaire, et le polymère obtenu doit y être insoluble.

Les monomères sont de préférence présents dans le solvant de synthèse, avant polymérisation, à raison de 5-20% en poids du mélange réactionnel. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

Le stabilisant :

Les particules de polymère sont stabilisées en surface, au fur et à mesure de la polymérisation, grâce à un stabilisant qui peut être notamment un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ;

les polymères hydrocarbonés greffés avec une chaîne siliconée.

Conviennent également les copolymères greffés ayant par exemple un squelette insoluble de type polyacrylique avec des greffons solubles de type acide poly (12-hydroxy stéarique).

On peut également utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

Le stabilisant peut aussi être choisi parmi les copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou encore les alkyl ($C_2$-$C_{18}$) méthicones copolyol. On peut par exemple utiliser le diméthicone copolyol vendu sous la dénomination "DOW CORNING 3225C" par la société DOW CORNING, ou le lauryl méthicone copolyol vendu sous la dénomination "DOW CORNING Q2-5200 par la société "DOW CORNING".

Comme copolymères blocs greffés ou séquencés, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces copolymères séquencés, on peut citer les copolymères de type "dibloc" ou "tribloc" du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation d'au moins un monomère éthylénique, tels que l'éthylène, l'isobutylène, et d'au moins un bloc d'un polymère acrylique tel que le méthacrylate de méthyle, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique et d'au moins un bloc d'un polyéther tel qu'un polyoxyalkylène en C2-C18, en particulier polyoxyéthylène et/ou polyoxypropylène, on peut citer les copolymères bi- ou triséquencés polyoxyéthylène/polybutadiène ou polyoxyéthylène/polyisobutylène.

On peut également employer des copolymères de (méth)acrylates d'alkyl en $C_1$-$C_4$, et de (méth)acrylates d'alkyl en $C_8$-$C_{30}$. On peut en particulier citer le copolymère méthacrylate de stéaryle/méthacrylate de méthyle.

Lorsqu'on utilise un polymère statistique en tant que stabilisant, on le choisit de manière à ce qu'il possède une quantité suffisante de groupements le rendant soluble dans le solvant de synthèse envisagé.

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxy ($C_2$-$C_{18}$)alkylène et notamment polyoxypropyléné et/ou oxyéthyléné.

Lorsque la phase grasse liquide ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyl en $C_1$-$C_4$, et d'acrylates ou de méthacrylates d'alkyl en $C_8$-$C_{30}$,
- (c) les copolymères blocs greffés ou séquencés comprenant au moins un bloc résultant de la polymérisation d'au moins un monomère éthylénique, à liaisons éthyléniques conjuguées,

et au moins un bloc d'un polymère vinylique ou acrylique ou d'un polyéther ou d'un polyester, ou leurs mélanges.

On peut bien entendu utiliser des associations des stabilisants décrits précédemment.

De préférence, on utilise des polymères dibloc comme agent stabilisant.

Suivant l'application, on pourra choisir d'utiliser des dispersions de polymères filmifiables ou non filmifiables, dans des huiles volatiles ou non volatiles.

Les additifs :

La composition selon l'invention peut contenir, en outre, au moins une cire qui peut être choisie parmi les cires d'origine animale, les cires d'origine végétale ou les cires d'origine synthétique.

Les cires susceptibles d'être utilisées dans la composition selon l'invention possèdent en règle générale un point de fusion compris entre 40 et 110 °C . La cire peut également avoir une pénétrabilité à l'aiguille allant de 1 à 217. La pénétrabilité à l'aiguille des cires est déterminée selon la norme française NF T 60-123 ou la norme américaine ASTM D 1321, à la température de 25 °C. Selon ces normes, la pénétrabilité à l'aiguille est la mesure de la profondeur, exprimée en dixièmes de millimètre, à laquelle une aiguille normalisée, pesant 2,5 g disposée dans un équipage mobile pesant 97,5 g et placée sur la cire à tester, pendant 5 secondes, pénètre dans la cire.

Parmi les cires d'origine animale, on peut citer les cires d'abeille, les cires de lanoline et les cires d'insectes de Chine.

Parmi les cires d'origine végétale, on peut citer les cires de riz, les cires de Carnauba, de Candellila, d'Ouricurry, les cires de fibres de liège, les cires de canne à sucre, les cires du Japon, la cire de Sumac, la cire de coton.

Parmi les cires d'origine minérale, on peut citer les paraffines, les cires microcristallines, les cires de Montan et les ozokérites.

Parmi les cires d'origine synthétique, on peut utiliser notamment les cires de polyoléfine et notamment les cires de polyéthylène, les cires obtenues par la synthèse de Fischer et Tropsch, les copolymères cireux ainsi que leurs esters, les cires de silicone.

Il est également possible d'utiliser des huiles d'origine animale ou végétale hydrogénées qui répondent toujours aux deux caractéristiques physiques mentionnées précédemment.

Parmi ces huiles, on peut citer les huiles hydrogénées qui sont obtenues par hydrogénation catalytique de corps gras composés de chaîne grasse linéaire ou non en $C_8$-$C_{32}$, l'huile de tournesol hydrogénée, l'huile de ricin hydrogénée, l'huile de coprah hydrogénée, l'huile de jojoba hydrogénée, la lanoline hydrogénée et les huiles de palme hydrogénées. Les cires utilisables selon la présente invention sont de préférence solides et rigides à température inférieure à 50 °C.

La composition selon l'invention peut comprendre de 0 % à 30 % (notamment de 0,1 % à 30 %) en poids de cire, en poids par rapport au poids total de la composition, de préférence de 1 % à 25 % en poids.

De préférence, la composition selon l'invention peut comprendre :

- au moins une cire ayant une pénétrabilité à l'aiguille allant de 1 à 7, 5 (dite cire I), notamment en une teneur allant de 0,1 % à 20 % en poids par rapport au poids total de la composition, et
- au moins une cire ayant une pénétrabilité à l'aiguille supérieure à 7,5 et inférieure ou égale à 217 (dite cire II), notamment en une teneur allant de 0,1 % à 10 % en poids par rapport au poids total de la composition. Ce mélange de cire est notamment approprié lorsque la composition est destinée à être utilisée comme mascara ou eye-liner.

La composition peut aussi contenir un polymère solubilisé dans la phase grasse liquide, dit encore polymère liposoluble.

Comme polymère lipophile, on peut notamment citer les copolymères résultant de la copolyméisation d'au moins un ester vinylique et d'au moins un autre monomère qui peut être une oléfine, un alkylvinyléther ou un ester allylique ou méthallylique, comme décrits dans la demande FR-A-22622303, dont le contenu est incorporé dans la présente demande à titre de référence.

Comme polymères filmogènes liposolubles utilisables dans l'invention, on peut également citer les polyalkylènes et notamment les copolymères d'alcènes en $C_2$-$C_{20}$ comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Le polymère filmogène liposoluble peut être présent dans la composition en une teneur allant de 0,1 % à 15 % en poids, par rapport au poids total de la composition, et mieux de 2 % à 10 % en poids.

La composition peut également comprendre au moins une matière colorante comme les composés pulvérulents et/ou les colorants liposolubles, par exemple à raison de 0,01 à 40 % du poids total de la composition. Les composés pulvérulents peuvent être choisis parmi les pigments et/ou les nacres habituellement utilisés dans les compositions cosmétiques. Avantageusement, les composés pulvérulents représentent de 0,1 à 25 % du poids total de la composition et mieux de 1 à 20 %.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La composition peut également comprendre des charges qui peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de Nylon (Orgasol de chez Atochem), de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut comprendre, en outre, un agent épaississant de la phase grasse liquide. L'agent épaississant peut être choisi parmi les argiles organomodifiées qui sont des argiles traitées par des composés choisis notamment parmi les amines quaternaires, les amines tertiaires. Comme argiles organomodifiées, on peut citer les bentonites organomodifiées telles que celles vendues sous la dénomination "Bentone 34" par la société RHEOX, les hectorites organomodifiées telles que celles vendues sous la dénomination "Bentone 27", "Bentone 38" par la société RHEOX.

On peut également employer des silices traitées, des gommes de guar alkylées liposolubles, des polymères gélifiant d'huile comme les polymères tribloc ou en étoile résultant de la polymérisation ou copolymérisation d'au moins un monomère à liaison éthylénique du type Kraton.

L'agent épaississant peut être présent en une teneur allant de 0,1 % à 10 % en poids, par rapport au poids total de la composition.

La composition peut contenir, en outre, tout additif usuellement utilisés dans de telles compositions tels que les parfums, les conservateurs, les tensioactifs, les plastifiants, les sequestrants, les vitamines, les protéines, les céramides, les agents alcalinisants ou acidifiants, les émollients.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

Selon un mode de réalisation de l'invention, la composition peut être essentiellement anhydre, et peut contenir moins de 10 % d'eau par rapport au poids total de la composition. Elle peut alors se présenter sous forme de gel huileux ou de pâte.

L'invention est illustrée plus en détail dans les exemples suivants.

**Exemple 1 de dispersion de polymère :**

On a préparé une dispersion de copolymère non réticulé d'acrylate de méthyle et d'acide acrylique dans un rapport 85/15, dans de l'isododécane, selon la méthode de l'exemple 7 du document EP-A-749 747. On obtient ainsi une dispersion de particules de poly(acrylate de méthyle/acide acrylique) stabilisées en surface dans de l'isododécane par un copolymère dibloc séquencé polystyrène/copoly(éhylènepropylène) vendu sous le nom de KRATON G1701 (Shell), ayant un taux de matière sèche de 22,6% en poids et une taille moyenne des particules de 175 nm (polydispersité : 0,05) et une Tg de 20°C. Ce copolymère est filmifiable à température ambiante (25 °C).

**Exemple 2 de dispersion de polymère :**

On a préparé une dispersion de copolymère non réticulé d'acrylate de méthyle et d'acide acrylique dans un rapport 95/5, dans de l'isododécane, comme indiqué à l'exemple 1. Le taux de matière sèche est de 24,2 % et la taille moyenne des particules de 180 nm et la Tg de 20 °C. Ce copolymère est filmifiable à température ambiante (25 °C).

**Exemple 3 de dispersion de polymère :**

On a préparé une dispersion de copolymère non réticulé de méthacrylate de méthyle et d'acide acrylique dans un rapport 85/15, dans de l'isododécane, selon la méthode de l'exemple 1 du document EP-A-749 746, en remplaçant l'heptane par de l'isododécane. On obtient ainsi une dispersion de particules de poly(méthacrylate de méthyle/acide acrylique) stabilisées en surface dans de l'isododécane par un copolymère dibloc séquencé polystyrène/copoly(éhylènepropylène) vendu sous le nom de KRATON G1701 (Shell), ayant un taux de matière sèche de 25,5 % en poids et une taille moyenne des particules de 150 nm (polydispersité < 0,1) et une Tg de 100 °C. Ce copolymère est non filmifiable à température ambiante (25 °C).

**Exemple 4 de dispersion de polymère :**

On prépare une dispersion de polyméthacrylate de méthyle réticulé par du diméthacrylate d'éthylène glycol, dans de l'isododécane, selon la méthode de l'exemple 2 du document EP-A-749 746, en remplaçant L'ISOPAR L par de l'isododécane. On obtient ainsi une dispersion de particules de polyméthacrylate de méthyle stabilisées en surface dans de l'isododécane par un copolymère dibloc séquencé polystyrène/copoly(éhylène-propylène) vendu sous le nom de KRATON G1701 (Shell), ayant un taux de matière sèche de 19,7% en poids et une taille moyenne des particules de 135 nm (polydispersité : 0,05) et une Tg de 100°C. Ce copolymère est non filmifiable à température ambiante (25 °C).

**Exemples 5 à 12 selon l'invention :**

On a préparé des mélanges (exemples 5 à 12) de dispersion de polymère filmifiable et de polymère non filmifiable selon les compositions suivantes :

- Dispersion de polymère filmifiable de l'exemple 1 ou 2 (appelée dispersion F1 ou F2) :        x g
- Dispersion de polymère non filmifiable de l'exemple 2 ou 3 (appelée dispersion NF 3 ou NF 4 ) :        y g

Pour chaque composition, on a mesuré la brillance du film obtenu. La brillance a été mesurée pour un film humide déposé de 300 μm d'épaisseur déposé sur carte de contraste puis conservé pendant 24 h, dans une atmosphère à 50 % d'humidité relative, pendant 24 h. La mesure a été effectuée à l'aide d'un brillancemètre BYK-GARDNER avec un angle de faisceau lumineux de 60°.

**Exemples 13 à 17 comparatifs :**

On a également comparé la composition de l'exemple 9 à 5 compositions (exemples 13 à 17) similaires dans lesquelles le polymère non filmifiable a été remplacé par une quantité équivalente en matières sèches de charge. La comparaison a été faite sur les propriétés de brillance du film obtenu selon la méthode indiquée précédemment.

**Résultats :**

On a obtenu les résultats suivants :

| Exemple | 5 | 6 | 7 |
|---|---|---|---|
| Dispersion F 1 | X = 90 | X = 70 | X = 50 |
| Dispersion NF 4 | Y = 10 | Y = 30 | Y = 50 |
| Brillance | 84,7 | 77,3 | 74,2 |

| Exemple | 8 | 9 | 10 | 11 | 12 |
|---|---|---|---|---|---|
| Dispersion F 2 | X = 90 | X = 80 | X = 70 | X = 60 | X = 50 |
| Dispersion NF 3 | Y = 10 | Y = 20 | Y = 30 | Y = 40 | Y = 50 |
| Brillance | 85,3 | 79,9 | 65,5 | 64,0 | 60,8 |

| Exemple | 13 (HI) | 14 (HI) | 15 (HI) | 16 (HI) | 17 (HI) |
|---|---|---|---|---|---|
| Dispersion F 2 | X = 80 | X = 80 | X = 80 | X = 80 | X = 80 |
| Charge | C1 | C2 | C3 | C4 | C5 |
| Brillance | 3,2 | 6,2 | 2,3 | 1,0 | 2,6 |

HI : hors invention
C1 : poudre de nylon - ORGASOL 2002 Extra D de la société ATOCHEM
C2 : Microbilles de résine polydiméthylsilsesquioxane - TOSPEARL 145a de la société TOSHIBA SILICONE
C3 : poudre de polyéthylène - ACUMIST B-6 de la société ALLIED CHEMICAL
C4 : Copolymère diméthacrylate d'éthylène glycol/méthacrylate de lauryle - POLYTRAP 6603 de la société APS
C5 : Sphères de copolymère de diméthacrylate d'éthylène glycol et de méthacrylate de méthyle - MICROSPONGE SKIN OIL ADSORBER 5640 POWDER de la société APS

On constate d'après ces résultats que les compositions selon l'invention (exemples 5 à 12) permettent d'obtenir un film ayant une bonne brillance (supérieur à 60), tandis que les compositions ne comportant pas de polymère non filmifiable mais des charges (exemples 13 à 17) conduisent à un film mat (brillance inférieure à 10). Une composition ne contenant que du polymère non filmifiable ne forme pas de film cohésif.

**Exemples 18 à 22 comparatifs de mascara** :

On a préparé 4 mascaras (exemples 18 à 21) selon l'invention ayant les compositions suivantes :

- Dispersion de polymère filmifiable de l'exemple 2 (appelée dispersion F 2)       x g
- Dispersion de polymère non filmifiable de l'exemple 3 (appelée dispersion NF 3)       y g
- Paraffine       2,2 g
- Cire de carnauba       4,5 g
- Cire d'abeille       8,3 g
- Copolymère acétate de vinyle/stéarate d'allyle (65/35) (Mexomère PQ de CHIMEX)       2,2 g
- Polylaurate de vinyle (Mexomère PP de CHIMEX)       0,75 g
- Amidon de riz       0,85 g
- Bentonite       5,32 g
- Carbonate de propylène       1,74 g
- Pigments       4,6 g
- Conservateurs       qs
- Isododécane       qsp       100 g

| Exemple | 18 | 19 | 20 |
|---|---|---|---|
| x | 5 | 10 | 8 |
| y | 5 | 10 | 2 |

On a également préparé un mascara (exemple 22) ne faisant pas partie de l'invention de composition suivante :

- Dispersion de polymère filmifiable de l'exemple 2 (appelée dispersion F 2)       8 g
- Sphères de copolymère diméthacrylate d'éthylène glycol/méthacrylate de méthyle (Microsponge skin oil adsorber 5640 powder de la société APS)       2 g
- Paraffine       2,2 g
- Cire de carnauba       4,5 g
- Cire d'abeille       8,3 g
- Copolymère acétate de vinyle/stéarate d'allyle (65/35) (Mexomère PQ de CHIMEX)       2,2 g
- Polylaurate de vinyle (Mexomère PP de CHIMEX)       0,75 g
- Amidon de riz       0,85 g
- Bentonite       5,32 g
- Carbonate de propylène       1,74 g

- Pigments     4,6 g
- Conservateurs     qs
- Isododécane     qsp     100 g

Cette composition comprend donc l'association d'une dispersion de polymère filmifiable avec une charge à la place d'un polymère non filmifiable.

Pour chaque composition, on a mesuré la dureté du film obtenu, ainsi que les propriétés recourbantes selon les méthodes suivantes :

a) <u>Mesure de la dureté du film</u> :

La dureté du film est mesurée selon la norme ASTM D-43-66, ou la norme NF-T 30-016 (octobre 1981), à l'aide d'un pendule de Persoz.

Le film déposé sur le support doit avoir une épaisseur de 300 microns avant séchage.

Après séchage pendant 24 heures, à 30°C et sous une humidité relative de 50%, on obtient un film ayant une épaisseur d'environ 100 microns; on mesure alors sa dureté à 30°C et 50% d'humidité relative, exprimée en seconde.

b) <u>Mesure du recourbement</u> :

On utilise des éprouvettes de 6 cheveux de 11 mm de long.

On mesure le recourbement initial Ri des cheveux non maquillés en mesurant l'angle formé par les cheveux par rapport à l'horizontale (on prend la moyenne de 6 mesures).

On maquille ensuite les éprouvettes en appliquant la composition en 3 fois 10 passages de brosse, en attendant 2 minutes après 10 passages.

On mesure le recourbement Rm des cheveux immédiatement après le maquillage en mesurant à nouveau l'angle formé par les cheveux maquillés (moyenne de 6 mesures).

On détermine ensuite le recourbement R = Rm - Ri.

On a obtenu les résultats suivants :

| Exemple | 18 | 19 | 20 | 21 | 22 |
|---|---|---|---|---|---|
| **Dureté (s)** | 14,7 | 17,2 | 19,5 | 14,8 | 12,4 |
| **Recourbement (°)** | 6,0 | 7,6 | 7,2 | 7,9 | 3,8 |

On constate que les compositions selon l'invention (exemples 18 à 21) forment un film présentant une dureté supérieure à celle du film de l'exemple 22 ne comprenant pas de particules de polymère non filmifiable mais une charge (microsponge). De plus, le recourbement obtenu avec les exemples 18 à 21 selon l'invention ( variation d'angle s'au moins 6, 0 °) est bien supérieur à celui obtenu avec l'exemple 22 (variation d'angle de 3,8 °).

Les compositions selon l'invention présentent donc de bonnes propriétés de recourbement des fibres kératiniques et forment des films de meilleure dureté. Une composition ne contenant que du polymère non filmifiable associé à une charge ne forme pas de film cohésif et n'a donc pas une dureté satisfaisante pour être employé dans un mascara.

**Exemple 23 :**

On a préparé un fond de teint ayant la composition suivante :

- Dispersion de polymère filmifiable de l'exemple 1     72,6 g
- Dispersion de polymère non filmifiable de l'exemple 4     9,4 g
- Pigments     10 g
- Charges     9,4 g

On a obtenu un fond de teint qui s'applique facilement sur le visage et qui donne un maquillage résistant à l'eau, naturel, non collant et présentant de bonnes propriétés de non transfert.

**Revendications**

1. Composition à application topique comprenant, dans un milieu physiologiquement acceptable, une dispersion de particules d'un premier système polymérique filmifiable comprenant un premier polymère, stabilisées en surface dans une phase grasse liquide, caractérisée par le fait qu'elle comprend, de plus, une dispersion de particules d'un deuxième système polymérique non filmifiable comprenant un deuxième polymère, stabilisées en surface dans la phase grasse liquide.

2. Composition selon la revendication 1, caractérisée par le fait que le premier système polymérique a une température de transition vitreuse (Tg 1) inférieure ou égale à 40 °C.

3. Composition selon l'une quelconque des revendications précédentes, caractérisée par le fait que le premier système polymérique a une température de transition vitreuse (Tg 1) allant de - 100 °C à 40 °C, et mieux de - 10 °C à 30 °C.

4. Composition selon l'une quelconque des revendications précédentes, caractérisée par le fait que le deuxième système polymérique a une température de transition vitreuse (Tg 2) supérieure à 40 °C.

5. Composition selon l'une quelconque des revendications précédentes, caractérisée par le fait que le deuxième système polymérique a une température de transition vitreuse (Tg 2) supérieure à 40 °C et inférieure ou égale à 300 °C, et mieux allant de 45 °C à 150 °C.

6. Composition selon l'une quelconque des revendications précédentes, caractérisée par le fait que le premier et/ou deuxième polymères sont choisis parmi les polymères radicalaires, les polycondensats, les polymères d'origine naturelle et leurs mélanges.

7. Composition selon l'une quelconque des revendications précédentes, caractérisée par le fait que le premier et/ou le deuxième polymères sont choisis parmi les polyuréthannes, polyuréthannes-acryliques, polyurées, polyurée/ polyuréthannes, polyester-polyuréthannes, polyéther-polyuréthannes, polyesters, polyesters amides, polyesters à chaîne grasse, alkydes ; polymères ou copolymères acryliques et/ou vinyliques ; polymères siliconés, polymères fluorés et leurs mélanges.

8. Composition selon l'une quelconque des revendications précédentes, caractérisée par le fait que le premier et/ou le deuxième système polymérique comprennent au moins un agent plastifiant.

9. Composition selon l'une quelconque des revendications précédentes, caractérisée par le fait que les particules de chaque système polymérique sont stabilisées notamment par un stabilisant choisi parmi les polymères séquencés, les polymères greffés, les polymères statistiques et leurs mélanges.

10. Composition selon la revendication 9, dans laquelle le stabilisant est choisi parmi les polymères siliconés greffés avec une chaîne hydrocarbonée ; les polymères hydrocarbonés greffés avec une chaîne siliconée ; les copolymères blocs greffés ou séquencés comprenant au moins un bloc de type polyorganosiloxane et au moins un bloc d'un polymère radicalaire ; les copolymères blocs greffés ou séquencés comprenant au moins un bloc de type polyorganosiloxane et au moins un bloc d'un polyéther ; les copolymères de (méth)acrylates d'alkyl en $C_1$-$C_4$ et de (méth)acrylates d'alkyl en $C_8$-$C_{30}$; les copolymères blocs greffés ou séquencés comprenant au moins un bloc résultant de la polymérisation de monomères éthyléniques comportant une ou plusieurs liaisons éthylénique éventuellement conjuguées et au moins un bloc d'un polymère styrénique ; les copolymères blocs greffés ou séquencés comprenant au moins un bloc résultant de la polymérisation de monomères éthyléniques et au moins un bloc d'un polymère acrylique ; les copolymères blocs greffés ou séquencés comprenant au moins un bloc résultant de la polymérisation de monomère éthylénique et au moins un bloc d'un polyéther, et leurs associations.

11. Composition selon la revendication 9 ou 10, caractérisée en ce que le stabilisant est un polymère bloc greffé ou séquencé, comprenant au moins un bloc résultant de la polymérisation de monomères éthyléniques comportant une ou plusieurs liaisons éthylénique éventuellement conjuguées et au moins un bloc d'un polymère styrénique.

12. Composition selon l'une quelconque des revendications précédentes, caractérisée par le fait que les premier et deuxième polymères dispersés dans la phase grasse liquide sont présents en une teneur totale allant de 2 % à 60 % en poids, par rapport au poids total de la composition, de préférence de 4 % à 40 % en poids, et mieux de 5 % à 30 % en poids.

**13.** Composition selon l'une quelconque des revendications précédentes, caractérisée par le fait que les premier et deuxième polymères sont présents dans la composition selon un rapport pondéral premier polymère/deuxième polymère allant de 1/99 à 99/1, de préférence de 50/50 à 99/1, et mieux de 50/50 à 80/20.

**14.** Composition selon l'une quelconque des revendications précédentes, caractérisée par le fait que la phase grasse liquide comprend au moins une huile choisie parmi les huiles d'origine minérale, animale, végétale ou synthétique, hydrocarbonées, fluorées et/ou siliconées, seules ou en mélange.

**15.** Composition selon l'une des revendications précédentes, dans laquelle la phase grasse liquide est choisie parmi l'huile de paraffine ou de vaseline, l'huile de vison, de tortue, de soja, le perhydrosqualène, l'huile d'amande douce, de calophyllum, de palme, de parléam, de pépins de raisin, de sésame, de maïs, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyldécyle, le palmitate de 2-octyl-décyle, le myristate ou le lactate de 2-octyldodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les PDMS éventuellement phénylés tels que les phényltriméthicones ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées ; les huiles volatiles telles que l'octaméthylcyclotétrasiloxane, le décaméthylcyclopentasiloxane, l'hexadéméthylcyclohexasiloxane, l'heptaméthylhexyltrisiloxane, l'heptaméthyloctyltrisiloxane ou les isoparaffines en $C_8$-$C_{16}$, et notamment l'isodo-décane, et leurs mélanges.

**16.** Composition selon l'une quelconque des revendications précédentes, caractérisée par le fait que la phase grasse liquide est choisie dans le groupe comprenant :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges.

**17.** Composition selon l'une quelconque des revendications précédentes, caractérisée en ce que la phase grasse liquide contient au moins une huile volatile à température ambiante.

**18.** Composition selon l'une quelconque des revendications précédentes, caractérisée par le fait qu'elle comprend, en outre, au moins une cire.

**19.** Composition selon l'une quelconque des revendications précédentes, caractérisée par le fait qu'elle comprend, en outre, au moins un polymère liposoluble.

**20.** Composition selon l'une quelconque des revendications précédentes, caractérisée par le fait que la composition comprend au moins un additif choisi dans le groupe formé par les agents épaississants de la phase grasse liquide, les parfums, les conservateurs, les tensioactifs, les sequestrants, les vitamines, les protéines, les céramides, les agents alcalinisants ou acidifiants, les émollients, les matières colorantes, les charges.

**21.** Composition selon l'une quelconque des revendications précédentes, caractérisée par le fait que la composition est essentiellement anhydre.

**22.** Composition selon l'une quelconque des revendications précédentes, caractérisée par le fait que la composition se présente sous la forme de mascara, d'eye-liner, de produit pour les lèvres, de fard à joues ou à paupières, de fond de teint, de produit de maquillage du corps, de produit anti-cernes, de base de maquillage, de composition à appliquer sur un maquillage, de composition de protection solaire, de coloration de la peau ou de produit de soin de la peau.

23. Procédé cosmétique de maquillage ou de traitement non thérapeutique des matières kératiniques, caractérisé par le fait que l'on applique sur les matières kératiniques une composition selon l'une quelconque des revendications 1 à 22.

24. Utilisation d'une composition telle que définie selon l'une quelconque des revendications 1 à 22 pour l'obtention d'un film déposé sur les matières kératiniques, notamment les fibres kératiniques, brillant et/ou non collant et/ou résistant aux frottements et/ou ayant des propriétés de non transfert.

25. Utilisation d'une composition telle que définie selon l'une quelconque des revendications 1 à 22 pour recourber les fibres kératiniques, notamment les cils.

**Office européen des brevets**

**RAPPORT DE RECHERCHE EUROPEENNE**

Numéro de la demande

EP 00 40 2259

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y | FR 2 772 601 A (L'OREAL) 25 juin 1999 (1999-06-25) * exemple 3 * --- | 1 | A61K7/032 A61K7/021 |
| A | EP 0 923 927 A (L'OREAL) 23 juin 1999 (1999-06-23) * exemple 5 * --- | 1 | |
| Y | EP 0 749 746 A (L'OREAL) 27 décembre 1996 (1996-12-27) * le document en entier * --- | 1 | |
| A | WO 98 42298 A (AVON PRODUCTS, INC.) 1 octobre 1998 (1998-10-01) * revendication 1 * --- | 1 | |
| A | WO 98 31329 A (L'OREAL) 23 juillet 1998 (1998-07-23) * revendication 1 * --- | 1 | |
| A | WO 97 01321 A (REVLON CONSUMER PROD. CORP.) 16 janvier 1997 (1997-01-16) * revendication 1 * ----- | 1 | **DOMAINES TECHNIQUES RECHERCHES (Int.Cl.7)** A61K |

Le présent rapport a été établi pour toutes les revendications

| Lieu de la recherche | Date d'achèvement de la recherche | Examinateur |
|---|---|---|
| LA HAYE | 27 novembre 2000 | Glikman, J-F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 .......................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 00 40 2259

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

27–11–2000

| Document brevet cité au rapport de recherche | | Date de publication | Membre(s) de la famille de brevet(s) | | Date de publication |
|---|---|---|---|---|---|
| FR 2772601 | A | 25–06–1999 | BR 9805529 | A | 11–04–2000 |
| | | | CN 1225257 | A | 11–08–1999 |
| | | | EP 0930060 | A | 21–07–1999 |
| | | | JP 11246441 | A | 14–09–1999 |
| | | | PL 330457 | A | 05–07–1999 |
| EP 923927 | A | 23–06–1999 | FR 2772603 | A | 25–06–1999 |
| | | | BR 9805553 | A | 11–04–2000 |
| | | | CN 1225259 | A | 11–08–1999 |
| | | | JP 11236313 | A | 31–08–1999 |
| | | | PL 330454 | A | 05–07–1999 |
| EP 749746 | A | 27–12–1996 | FR 2735691 | A | 27–12–1996 |
| | | | FR 2735690 | A | 27–12–1996 |
| | | | FR 2735692 | A | 27–12–1996 |
| | | | FR 2735684 | A | 27–12–1996 |
| | | | AT 157529 | T | 15–09–1997 |
| | | | DE 69600059 | D | 09–10–1997 |
| | | | DE 69600059 | T | 05–02–1998 |
| | | | DK 749746 | T | 23–02–1998 |
| | | | ES 2110857 | T | 16–02–1998 |
| | | | WO 9700662 | A | 09–01–1997 |
| | | | GR 3025474 | T | 27–02–1998 |
| | | | JP 3027008 | B | 27–03–2000 |
| | | | JP 10502389 | T | 03–03–1998 |
| | | | KR 231637 | B | 15–11–1999 |
| | | | US 5945095 | A | 31–08–1999 |
| WO 9842298 | A | 01–10–1998 | AU 6548798 | A | 20–10–1998 |
| | | | EP 1005322 | A | 07–06–2000 |
| | | | US 6083516 | A | 04–07–2000 |
| WO 9831329 | A | 23–07–1998 | CA 2249478 | A | 23–07–1998 |
| | | | EP 0909157 | A | 21–04–1999 |
| | | | US 6126929 | A | 03–10–2000 |
| WO 9701321 | A | 16–01–1997 | AU 6286296 | A | 30–01–1997 |
| | | | CA 2225996 | A | 16–01–1997 |
| | | | EP 0835091 | A | 15–04–1998 |
| | | | US 6143283 | A | 07–11–2000 |
| | | | US 5849275 | A | 15–12–1998 |

EPO FORM P0460

Pour tout renseignement concernant cette annexe : voir Journal Officiel de l'Office européen des brevets, No.12/82